# EUROPÄISCHE PATENTSCHRIFT

(11) **EP 1 748 809 B1**
(45) Veröffentlichungstag und Bekanntmachung des Hinweises auf die Patenterteilung: **05.11.2008**
(21) Anmeldenummer: 05741232.2
(22) Anmeldetag: 10.05.2005
(51) Int. Cl.: A61M 1/10

(54) **VORRICHTUNG ZUR EPIKARDIALEN UNTERSTÜTZUNG UND/ODER ÜBERNAHME DER HERZTÄTIGKEIT**
DEVICE FOR THE EPICARDIAL SUPPORT AND/OR RESUMPTION OF CARDIAC ACTIVITY
DISPOSITIF D'ASSISTANCE EPICARDIQUE ET/OU DE REPRISE DE L'ACTIVITE CARDIAQUE

(30) Priorität: 11.05.2004 DE 102004023191
(43) Veröffentlichungstag der Anmeldung: 07.02.2007
(73) Patentinhaber: PPA Technologies AG, 07745 Jena (DE)
(72) Erfinder: FERRARI, Markus, 07747 Jena (DE)
(74) Vertreter: Weidener, Jörg Michael
(86) Internationale Anmeldenummer: PCT/EP2005/005053
(87) Internationale Veröffentlichungsnummer: WO 2005/110514

(56) Entgegenhaltungen:
- WO-A-95/18593
- DE-A1- 19 951 220
- US-A- 3 513 836
- US-A- 5 738 627

## Beschreibung

Die vorliegende Erfindung betrifft eine Vorrichtung zur epikardialen Unterstützung und/oder Übernahme der Herztätigkeit, mit einer Doppelmembran, die eine elastische innere Membran und eine nichtdehnbare äußere Membran sowie einen dazwischen gebildeten, geschlossenen und mittels eines Fluids inflatierbaren und deflatierbaren Hohlraum aufweist, wobei ein zweiter Hohlraum mit einer den zweiten Hohlraum begrenzenden Wandung vorgesehen ist, der über eine separate Fluidleitung von außerhalb des Patientenkörpers mit einem Fluid befüllbar oder darin befindliches Fluid nach außen ableitbar ist.

Eine Vorrichtung der eingangs genannten Art ist bereits aus der WO-A-95/18593 bekannt. Es ist auch bekannt, daß Herzpatienten nach einem operativen Eingriff zur Kräftigung des Herzens medikamentöse Unterstützung benötigen. Die entsprechenden Medikamente werden dabei üblicherweise über einen gesondert zu legenden Herzkatheter in den Herzbeutel infundiert. Dies bedeutet jedoch einen zusätzlichen Aufwand mit einem entsprechenden Risiko für den Patienten.

Weiterhin ist aus der DE-A-199 51 220 eine Vorrichtung zur Unterstützung und/oder Übernahme der Herztätigkeit bekannt. Es handelt sich dabei um ein wenig invasives, das heißt perkutan implantierbares System zur mechanischen Unterstützung und zum temporären Ersatz der Pumpfunktion des Herzens. Die Vorrichtung wird nach Sondierung des Herzbeutels in zusammengefaltetem Zustand perkutan in den Herzbeutel eingebracht oder am Ende einer Operation in den Herzbeutel chirurgisch positioniert und dort mit der Doppelmembran um die rechte und linke Herzkammer gelegt. Dabei ist die Vorrichtung in deflatiertem Zustand so dünn, daß eine Kompression der Nachbarorgane vermieden wird. Nach der Implantation wird der Hohlraum der Doppelmembran über einen Verbindungsschlauch rhythmisch mit einem Fluid beaufschlagt, welches entweder ein Gas (Helium oder CO₂) oder eine geeignete Flüssigkeit sein kann. Durch dieses rhythmische Inflatieren und Deflatieren des Hohlraums der Doppelmembran und weil die äußere Membran im Gegensatz zur inneren Membran nicht dehnbar ist, kommt es zu einer Druckübertragung und Kompression des Herzens über die das Herz umschließende Doppelmembran. Dabei wird das Blut aus der rechten Herzkammer in die Pulmonalarterie und gleichzeitig aus der linken Kammer in die Aorta ausgetrieben oder bei vorhandener Pumpfunktion des Herzens die systolische Auswurfarbeit des Herzmuskels unterstützt. Auch bei dieser bekannten Vorrichtung erfolgt nach einem operativen Eingriff eine medikamentöse Unterstützung des Herzens über einen gesondert zu legenden Herzkatheter mit den zuvor genannten Nachteilen.

Aufgabe der vorliegenden Erfindung ist es, eine Vorrichtung der eingangs genannten Art zur Verfügung zu stellen, die eine einfache und risikoarme Applikation von Medikamenten ermöglicht.

Die vorgenannte Aufgabe ist bei einer Vorrichtung der eingangs genannten Art erfindungsgemäß dadurch gelöst, daß sich der zweite Hohlraum auf der dem Herzen zugewandten Innenseite der inneren Membran befindet und daß die herzseitige Wandung des zweiten Hohlraums fluiddurchlässig ist.

Die Vorteile der vorliegenden Erfindung liegen insbesondere darin, daß somit nicht nur Medikamente ohne zusätzlichen Aufwand in den Herzbeutel infundiert, sondern ebenso Flüssigkeiten, wie z. B. Wundsekret, nach außen abgeleitet werden können. Diese erfindungsgemäße Weiterbildung der aus der Druckschrift WO-A-95/18593 bekannten Vorrichtung ist insofern von großem Vorteil, da die Vorrichtung an sich bereits um den Herzbeutel gelegt, also implantiert wurde. Insofern erübrigt sich das zusätzliche und bislang erforderliche Legen eines Herzkatheters. Darüber hinaus ist es von immensem Vorteil, daß die Medikamentenapplikation durch die erfindungsgemäße Vorrichtung sehr breitfächig, gewünschtenfalls über die gesamte durch die Doppelmembran bedeckte Herzoberfläche, erfolgen kann. Schließlich ist es von großem Vorteil, daß die Medikamentenapplikation über die separate Fluidleitung ortsunabhängig erfolgen kann, was insbesondere für die kritische postoperative Phase von Interesse ist.

Vorteilhafte Weiterbildungen der Erfindung sind in den Unteransprüchen 2 bis 5 angegeben.

Zur Ausbildung des zweiten Hohlraums mit seiner herzseitigen fluiddurchlässigen Wandung sind zwei alternative Möglichkeiten vorgesehen: Zum einen kann der zweite Hohlraum herzseitig durch eine semipermeable Membran als fluiddurchlässige Wandung begrenzt sein. Der zweite Hohlraum wird dann zwischen der elastischen inneren Membran der Doppelmembran und jener herzseitig dazu angeordneten semipermeablen Membran gebildet. Dabei definiert dann die Porengröße der semipermeablen Membran die Molekülgröße und die Menge der zu applizierenden Medikamentensubstanz.

Alternativ dazu kann der zweite Hohlraum durch eine auf der herzseitigen Innenseite der inneren Membran angeordnete Leitung mit definierten, zum Herz weisenden Öffnungen gebildet sein. Selbstverständlich können auf der herzseitigen Innenseite der inneren Membran mehrere solcher Leitungen 10 angeordnet sein, um eine möglichst flächendeckende Applikation auf den Herzmuskel zu erzielen. Dabei sind die Leitungen mit Dränagerohren vergleichbar, die ja bekanntlich auch definierte Öffnungen aufweisen.

Da nach koronaren Bypass-Operationen eine externe Kompression der epikardialen Gefäße nicht wünschenswert ist, weist die Doppelmembran im Bereich der großen Herzkranzgefäße vorzugsweise variable Aussparungen auf. Eine solche Doppelmembran kann entweder individuell auf den Patienten angepasst angefertigt oder aber, wie gemäß einer weiteren vorteilhaften Weiterbildung vorgesehen, mittels verschiebbarer Abstützungen an die besonderen Erfordernisse eines Patientenherzens angepasst werden. Dabei können die variablen Aussparungen durch faltbare, flexible Stege oder Halbschläuche durch mechanische Manipulation des Chirurgen in die gewünschte Position gebracht werden. Diese faltbaren, flexiblen Stege oder Halbschläuche können während des Pumpbetriebes entweder durch selbsthaftende Eigenschaften, Anwendung eines Gewebe-Klebstoffes, durch eine Halteschiene, oder durch Rillen innerhalb der Doppelmembran, welche bestimmte Positionen vorgeben, in der gewünschten Position gehalten werden.

Im folgenden wird ein Ausführungsbeispiel der Erfindung anhand einer Zeichnung näher erläutert.

Es zeigen:
- Figur 1:: eine schematische Darstellung der erfindungsgemäßen Vorrichtung;
- Figur 2:: eine schematische Darstellung der erfindungsgemäßen Vorrichtung mit einer vergrößerten Ausschnittsdarstellung eines zweiten Hohlraums mit einer semipermeablen Membran;
- Figur 3:: eine mit der Figur 2 vergleichbare Darstellung, wobei die ausschnittsweise Vergrößerung rechts oben im Bild diesmal einen durch eine Leitung gebildeten zweiten Hohlraum zeigt; und
- Figur 4:: die Darstellung eines menschlichen Herzens mit aufgesetzter Vorrichtung und Aussparungen.

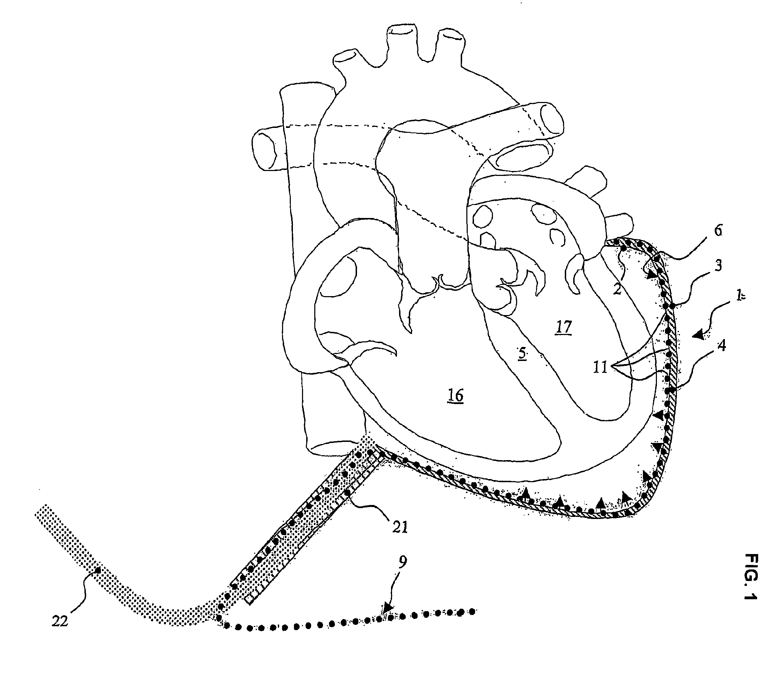

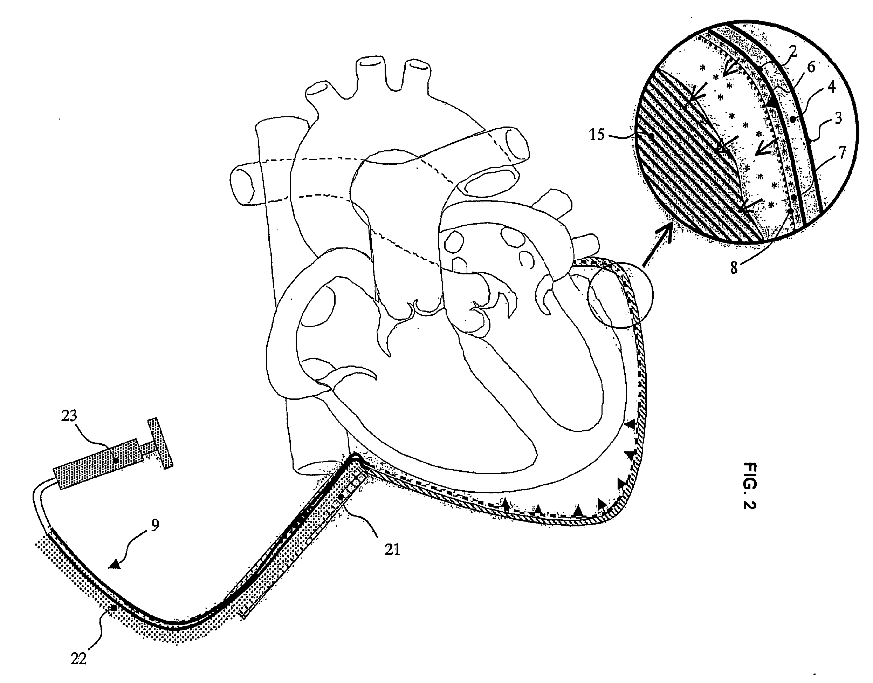

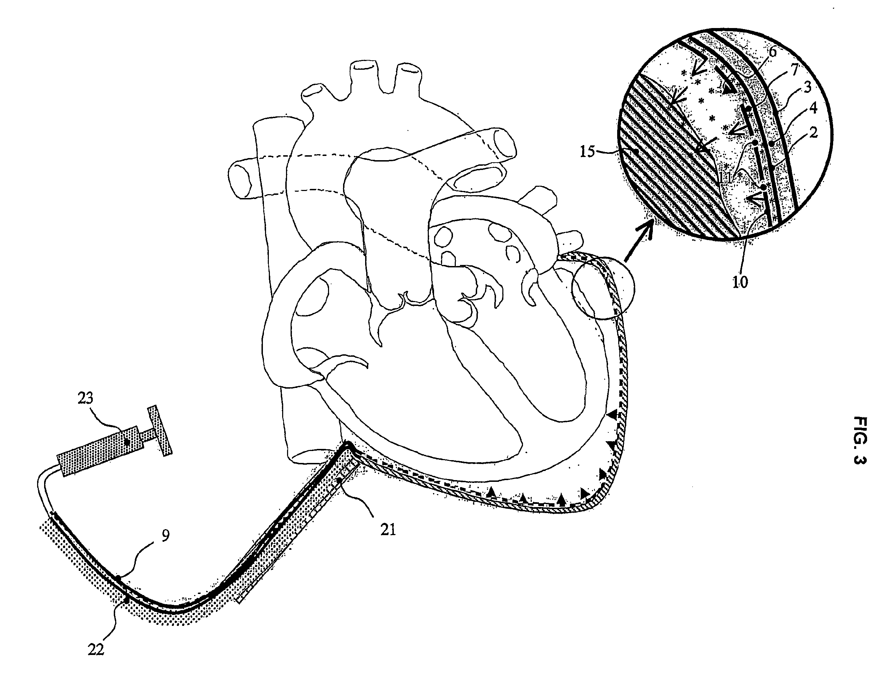

Figur 1 zeigt eine schematische Darstellung einer Vorrichtung zur epikardialen Unterstützung und/oder Übernahme der Herztätigkeit, mit einer Doppelmembran 1, die eine elastische innere Membran 2 und eine nicht dehnbare äußere Membran 3 sowie einen dazwischen gebildeten, geschlossenen und mittels eines Fluids infaltierbaren und deflatierbaren Hohlraum 4 aufweist. Auf der dem Herzen 5 zugewandten Innenseite 6 der inneren Membran 2 ist eine durch eine punktierte Line angedeutete Medikamentenapplikation dargestellt, die durch Öffnungen 11 in einer fluiddurchlässigen Wandung (vergleiche Figuren 2 und 3) erfolgt. Die Medikamentenzufuhr erfolgt von außen mittels einer separaten Fluidleitung 9 durch eine Einführschleuse 21 durch die Haut neben dem Brustbein des Patienten. Ebenso durch diese Einführschleuse 21 verläuft der Fluidschlauch 22 zum Inflatieren bzw. Deflatieren des Hohlraums 4 der Doppelmembran 1.

Figur 2 zeigt eine ähnlich schematische Darstellung der erfindungsgemäßen Vorrichtung mit einer Ausschnittsvergrößerung der Doppelmembran 1 in der rechten oberen Ecke der Figur 2. Diese Ausschnitssvergrößerung zeigt, dass sich an die Innenseite 6 der inneren Membran 2 ein zweiter Hohlraum 7 anschließt, der herzseitig durch eine fluiddurchlässige Wandung in Form einer semipermeablen Membran 8 begrenzt ist. Dieser zweite Hohlraum 7 kann von außerhalb des Patienten über eine Infusionspumpe 23 und eine separate Fluidleitung 9 zur Infusion von Medikamenten in den Herzbeutel benutzt werden. Dabei definiert die Porengröße der semipermeablen Membran 8 die Molekülgröße und Menge der zu applizierenden Substanz, welche durch die kleinen Sternchen in dem Hohlraum 7 und im Bereich des Herzmuskels 15 angedeutet ist.

Figur 3 zeigt eine mit der Figur 2 vergleichbare Darstellung, wobei hier allerdings die Ausschnittsvergrößerung in der rechten oberen Ecke des Bildes einen durch eine oder mehrere Leitungen 10 mit definierten Öffnungen 11 gebildeten Hohlraum 7 aufweist. Auch in diesem Fall wird dem Hohlraum 7, also der bzw. den Leitung(en), das Medikament - wieder durch kleine Sternchen angedeutet - über eine Infusionspumpe 23 und eine separate Fluidleitung 9 zugeführt. Dabei ist die zugeführte Medikamentenmenge abhängig vom Infusionsdruck und der Größe der definierten Öffnungen.

Selbstverständlich ist es bei beiden Ausführungsformen des zweiten Hohlraums 7, also sowohl dann, wenn er durch eine semipermeable Membran 8 gebildet ist, als auch dann, wenn er durch eine oder mehrere Leitungen 10 mit definierten, zum Herzen weisenden Öffnungen 11 gebildet ist, möglich, nicht nur Medikamente enthaltendes Fluid zuzuführen, sondern beispielsweise auch Fluid in Form von Wundsekret nach außen abzuleiten.

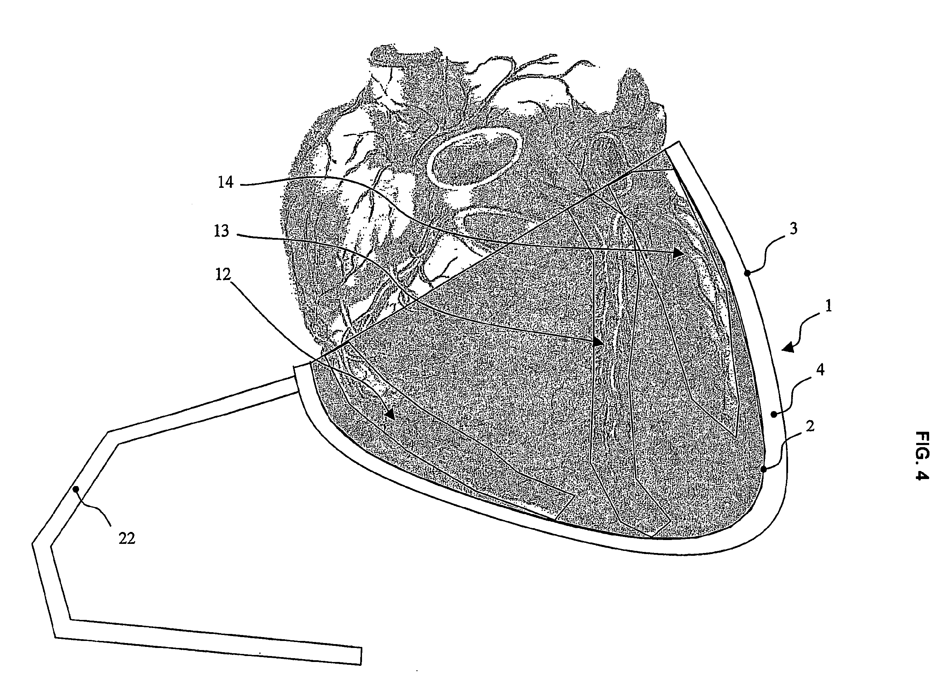

Figur 4 zeigt ein Patientenherz mit einer schematisch darumgelegten Doppelmembran 1, deren Hohlraum 4 wiederum über den Fluidschlauch 22 inflatierbar und deflatierbar ist. Dieses Ausführungsbeispiel der Doppelmembran 1 weist im Bereich der großen Herzkranzgefäße variable Aussparungen 12, 13, 14 auf, um eine externe Kompression der epikardialen Gefäße zu vermeiden. Diese Aussparungen 12, 13, 14 sind mittels nicht dargestellter verschiebarer Abstützungen an die Erfordernisse eines Patientenherzens anpassbar.

## Patentansprüche

1. Vorrichtung zur epikardialen Unterstützung und/oder Übernahme der Herztätigkeit, mit einer Doppelmembran (1), die eine elastische innere Membran (2) und eine nichtdehnbare äußere Membran (3) sowie einen dazwischen gebildeten, geschlossenen und mittels eines Fluids inflatierbaren und deflatierbaren Hohlraum (4) aufweist, wobei ein zweiter Hohlraum mit einer den zweiten Hohlraum begrenzenden Wandung vorgesehen ist, der über eine separate Fluidleitung (9) von außerhalb des Patientenkörpers mit einem Fluid befüllbar oder darin befindliches Fluid nach außen ableitbar ist,
**dadurch gekennzeichnet, daß**
sich der zweite Hohlraum (7) auf der dem Herzen (5) zugewandten Innenseite (6) der inneren Membran (2) befindet und daß die herzseitige Wandung des zweiten Hohlraums (7) fluiddurchlässig ist.

2. Vorrichtung nach Anspruch 1,
**dadurch gekennzeichnet, daß**
der zweite Hohlraum (7) herzseitig durch eine semipermeable Membran (8) begrenzt ist.

3. Vorrichtung nach Anspruch 1,
**dadurch gekennzeichnet, daß**
der zweite Hohlraum (7) durch eine auf der herzseitigen Innenseite (6) der inneren Membran (2) angeordnete Leitung (10) mit definierten, zum Herzen weisenden Öffnungen (11) gebildet ist.

4. Vorrichtung nach einem der vorstehenden Ansprüche,
**gekennzeichnet durch**
Aussparungen (12, 13, 14) in der Doppelmembran (1) im Bereich der großen Herzkranzgefäße.

5. Vorrichtung nach Anspruch 4,
**gekennzeichnet durch**
verschiebbare Abstützungen, mittels derer die Aussparungen (12, 13, 14) an die Erfordernisse eines Patientenherzens anpaßbar sind.

## Claims

1. Device for epicardial support and/or the assuming of cardiac activity having a double membrane (1) consisting of an elastic inner membrane (2) and a non-expandable outer membrane (3) as well as a closed cavity (4) formed therebetween which can be inflated and deflated by means of a fluid, wherein a second cavity having a wall limiting the second cavity is provided, which can be filled with a fluid by a separate fluid line from out side of the patient's body, or fluid from within can be drained out,
**characterized in that**
the second cavity (7) is provided at the inner side (6) of the inner membrane (2) facing inwardly to the heart (5) and that the heart facing wall of the second cavity (7) is fluid permeable.

2. Device according to claim 1,
**characterized in that**
the second cavity (7) is limited respective the heart by a semi-permeable membrane (8).

3. Device according to claim 1,
**characterized in that**
the second cavity (7) is formed by a line (10) arranged on the inward heart-facing side (6) of the inner membrane (2) with defined openings (11) to the heart.

4. Device according to any one of the preceding claims,
**characterized in that**
recesses (12, 13, 14) are provided in the double membrane (1) in the area of the large coronary artery.

5. Device according to claim 4,
**characterized in that**
displaceable supports are provided by means of which the recesses (12, 13, 14) can be adapted to the requirements of a patient's heart.

## Revendications

1. Dispositif d'assistance épicardique et/ou de reprise de l'activité cardiaque, comprenant une double membrane (1) qui présente une membrane interne élastique (2) et une membrane externe non élastique (3), ainsi qu'une cavité (4) fermée formée entre elles, pouvant être gonflée et dégonflée au moyen d'un fluide, une deuxième cavité étant prévue avec une paroi limitant la deuxième cavité, qui peut être remplie par le biais d'une conduite de fluide séparée (9) depuis l'extérieur du corps du patient, avec un fluide, ou du fluide s'y trouvant pouvant être évacué vers l'extérieur,
**caractérisé en ce que**
la deuxième cavité (7) se trouve du côté intérieur (6) de la membrane interne (2) tourné vers le coeur (5), et **en ce que** la paroi de la deuxième cavité (7), du côté du coeur, est perméable aux fluides.

2. Dispositif selon la revendication 1,
**caractérisé en ce que**
la deuxième cavité (7) du côté du coeur est limitée par une membrane semi-perméable (8).

3. Dispositif selon la revendication 1,
**caractérisé en ce que**
la deuxième cavité (7) est formée par une conduite (10) disposée du côté intérieur (6) du côté du coeur de la membrane interne (2), avec des ouvertures (11) définies, tournées vers le coeur.

4. Dispositif selon l'une quelconque des revendications précédentes,
**caractérisé par**
des évidements (12, 13, 14) dans la double membrane (1) dans la région des gros vaisseaux coronariens.

5. Dispositif selon la revendication 4,
**caractérisé par**
des supports déplaçables au moyen desquels les évidements (12, 13, 14) peuvent être adaptés aux besoins du coeur d'un patient.
